(19) Europäisches Patentamt
European Patent Office
Office européen des brevets

(11) **EP 4 198 933 B1**

(12) **EUROPEAN PATENT SPECIFICATION**

(45) Date of publication and mention
of the grant of the patent:
**15.10.2025 Bulletin 2025/42**

(21) Application number: **22747567.0**

(22) Date of filing: **21.03.2022**

(51) International Patent Classification (IPC):
*G08B 21/04* *(2006.01)*

(52) Cooperative Patent Classification (CPC):
**G08B 21/043; A61B 5/1038; A61B 5/1117;
A61B 5/112; A61B 5/7275;** A61B 2562/0219

(86) International application number:
**PCT/KR2022/003882**

(87) International publication number:
**WO 2023/075042 (04.05.2023 Gazette 2023/18)**

(54) **FALL RISK PREVENTION METHOD AND DEVICE FOR CARRYING OUT SAME**

VERFAHREN ZUR STURZRISIKOVERHINDERUNG UND VORRICHTUNG ZUR DURCHFÜHRUNG DES VERFAHRENS

PROCÉDÉ DE PRÉVENTION DES RISQUES DE CHUTE ET DISPOSITIF POUR SA MISE EN OEUVRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.10.2021 KR 20210142549**

(43) Date of publication of application:
**21.06.2023 Bulletin 2023/25**

(73) Proprietor: **Welt Corp., Ltd
Seoul 06628 (KR)**

(72) Inventors:
• **KANG, Sung Ji
Gangnam-gu, Seoul 06366 (KR)**
• **ROH, Hye Gang
Songpa-gu, Seoul 05507 (KR)**
• **JUNG, Hwa Young
Namyangju-si, Gyeonggi-do 12249 (KR)**

(74) Representative: **AWA Sweden AB
Box 5117
200 71 Malmö (SE)**

(56) References cited:
JP-A- 2019 150 329      KR-A- 20100 004 967
KR-A- 20180 023 225     KR-A- 20190 070 068
KR-A- 20210 115 450     US-A1- 2016 100 776
US-A1- 2018 177 436

**Description**

BACKGROUND

**1. Field of the Invention**

The present invention relates to a method of preventing a fall risk and to a fall prevention apparatus for preventing a fall risk.

**2. Discussion of Related Art**

With the development of various smart technologies, data on personal daily activities can be recorded and personal life can be managed more efficiently based on the recorded data. Amid this, interest in health is increasing, and thus health-related data logging is being focused on. Many users are already generating and utilizing various pieces of health-related data, such as the users' exercise, diet, sleep, and the like, through user devices such as smartphones and wearable devices. In the past, health-related data was generated and managed only by medical institutions, whereas users have now started to generate and manage their own health-related data through user devices such as smartphones or wearable devices.

Health-related data logging is often performed through wearable devices. A wearable device is a user device that is carried or attached to a user's body. Wearable devices are being widely used to collect health-related data due to the development of the Internet of Things and the like. A wearable device can collect information on changes in a user's body and data on an environment surrounding the user through a device, and can provide advice necessary for the user's health on the basis of the collected data.

Currently, a procedure for providing feedback using health-related data acquired through a wearable device cannot be elaborated and thus cannot be utilized for specific medical practices. However, according to the development of user devices capable of collecting various pieces of health-related data in addition to the wearable devices and the refinement of decision algorithms based on the health-related data obtained through the user devices, the health-related data obtained through the user devices can be used for actual medical practices.

Health-related data logging also enables analysis of risks that can occur in a user's life. Specifically, it is possible to determine a fall risk through a wearable device and provide information on the fall risk beforehand to a user. There is a need for specific research and development on techniques for performing fall estimation based on data.

Patent application US 2016/0100776 A1 relates to a light-weight, small and portable ambulatory sensor for measuring and monitoring a person's physical activity.

Patent application US 2018/0177436 A1 relates to a system and method for remote monitoring for elderly fall prediction, detection, and prevention that includes collecting sensor data at a biomechanical sensing device coupled to a user.

SUMMARY OF THE INVENTION

The present invention is directed to solving all of the above problems.

Further, the present invention is directed to collecting gait data of a user and estimating a fall risk that may occur for the user to prevent a fall.

Further, the present invention is also directed to more accurately estimating a fall risk that may occur for a user in consideration of a gait speed of the user through preprocessing of gait data of the user.

A representative configuration of the present invention for achieving the above objects is as follows.

According to an aspect of the present invention, there is provided a method of preventing a fall risk, including receiving, by a fall prevention apparatus, gait data, generating, by the fall prevention apparatus, gait analysis data on the basis of analysis of the gait data, and generating, by the fall prevention apparatus, fall prevention data on the basis of the gait analysis data.

Meanwhile, analysis of the gait analysis data is performed based on a maximum peak and a minimum peak appearing in the gait data, and the gait analysis data includes data on a gait speed and balance of both feet.

Further, the gait speed is determined based on a step interval time determined based on the maximum peak, and the balance of both feet is determined using an impulse calculated based on an amplitude between the maximum peak and the minimum peak. Further, the maximum and minimum peaks are determined by using an erroneous detection removal operation to remove a fake peak from a candidate minimum peak and a candidate maximum peak, in which erroneous detection removal operation a minimum one gait period is set as a critical period and information on peaks generated within the critical period is removed after one peak is generated.

According to another aspect of the present invention, there is provided a fall prevention apparatus for preventing a fall risk, including a gait data input unit configured to receive gait data, a gait data analysis unit configured to generate gait analysis data on the basis of analysis of the gait data, and a fall prevention unit configured to generate fall prevention data on the basis of the gait analysis data.

Meanwhile, analysis of the gait analysis data is performed based on a maximum peak and a minimum peak appearing in the gait data, and the gait analysis data includes data on a gait speed and balance of both feet.

Further, the gait speed is determined based on a step length and a step interval time which are determined based on the maximum peak and the minimum peak, and the balance of both feet is determined using an impulse calculated based on an amplitude between the maximum peak and the minimum peak. Further, the maximum and minimum peaks are determined by using an erroneous detection removal operation to remove a fake peak from a candidate minimum peak and a candidate maximum peak, the erroneous detection removal operation comprises one gait period being set as a critical period and information on peaks generated within the critical period is removed after one peak is generated.

BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

FIG. 1 is a conceptual diagram showing a fall prevention apparatus according to an embodiment of the present invention;
FIG. 2 shows conceptual diagrams showing gait data according to an embodiment of the present invention;
FIG. 3 is a conceptual diagram showing an operation of a gait data analysis unit according to an embodiment of the present invention;
FIG. 4 shows conceptual diagrams showing the operation of the gait data analysis unit according to the embodiment of the present invention;
FIG. 5 shows conceptual diagrams showing the operation of the gait data analysis unit according to the embodiment of the present invention;
FIG. 6 shows conceptual diagrams showing an erroneous detection removal operation according to an embodiment of the present invention;
FIG. 7 is a conceptual diagram showing an additional peak detection operation according to an embodiment of the present invention;
FIG. 8 is a conceptual diagram showing a method of determining, by a gait data analysis unit, a gait speed according to an embodiment of the present invention;
FIG. 9 shows conceptual diagrams showing a method of determining, by a gait data analysis unit, an impulse according to the embodiment of the present invention;
FIG. 10 is a conceptual diagram showing a method of estimating, by a fall estimation unit, a fall according to an embodiment of the present invention; and
FIG. 11 shows conceptual diagrams showing a user screen for displaying results of estimating falls according to an embodiment of the present invention.

DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope of the present invention. In addition, it should be understood that a position or arrangement of each component in each disclosed embodiment may be changed without departing from the scope of the present invention. Accordingly, there is no intent to limit the present invention to the detailed description to be described below. The scope understood that various embodiments of the present invention are different but are not necessarily mutually exclusive. For example, a specific shape, structure, and characteristic of an embodiment described herein may be implemented in another embodiment without departing from the scope of the present invention. In addition, it should be understood that a position or arrangement of each component in each disclosed embodiment may be changed without departing from the scope of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

Hereinafter, in order to enable those skilled in the art to practice the present invention, exemplary embodiments of the present invention will be described in detail with reference to the accompanying drawings.

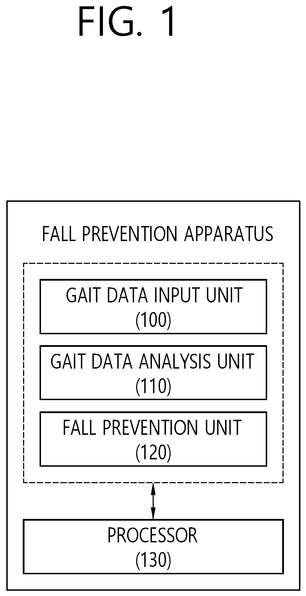

FIG. 1 is a conceptual diagram showing a fall prevention apparatus according to an embodiment of the present invention.

In FIG. 1, the fall prevention apparatus for analyzing the likelihood of a user falling through analysis of a gait of the user is disclosed.

Referring to FIG. 1, the fall prevention apparatus includes a gait data input unit 100, a gait data analysis unit 110, a fall prevention unit 120, and a processor 130.

The gait data input unit 100 is implemented to receive gait data. The gait data may be generated by a separate wearable device (e.g., a smart belt) and transmitted to the gait data input unit 100, or the gait data may be directly generated by the fall prevention apparatus and transmitted to the gait data input unit 100.

The gait data analysis unit 110 is implemented to analyze the input gait data. The gait data analysis unit 110 determines a gait step on the basis of analysis of the gait data and determine a gait speed on the basis of the gait step. Further, the gait data analysis unit 110 determines an impulse on the basis of the gait data and determine a balance of both feet on the basis of the impulse. That is, the gait data analysis unit 110 analyzes the gait data to determine the gait speed and the balance of both feet.

Specifically, the gait data analysis unit 110 may process the gait data by performing a preprocessing operation on gait data, a peak detection operation, an erroneous detection removal operation, and an additional peak detection operation, extract gait analysis data such as the gait speed and the balance of both feet appearing in the gait data, and transmit the gait analysis data to the fall prevention unit 120.

The fall prevention unit 120 is implemented to prevent falls on the basis of the gait analysis data. The fall prevention unit 120 generates fall prevention data on the basis of the gait analysis data. The fall prevention data may include data on the likelihood of the user falling and prevention data for avoiding falls.

The fall prevention data generated by the fall prevention unit 120 may be provided to the user to inform the user of a fall risk. For example, the fall prevention apparatus may transmit the fall prevention data to a user device such as a smartphone of the user so that the user prepares for a fall beforehand.

The processor 130 may be implemented to control the operations of the gait data input unit 100, the gait data analysis unit 110, and the fall prevention unit 120.

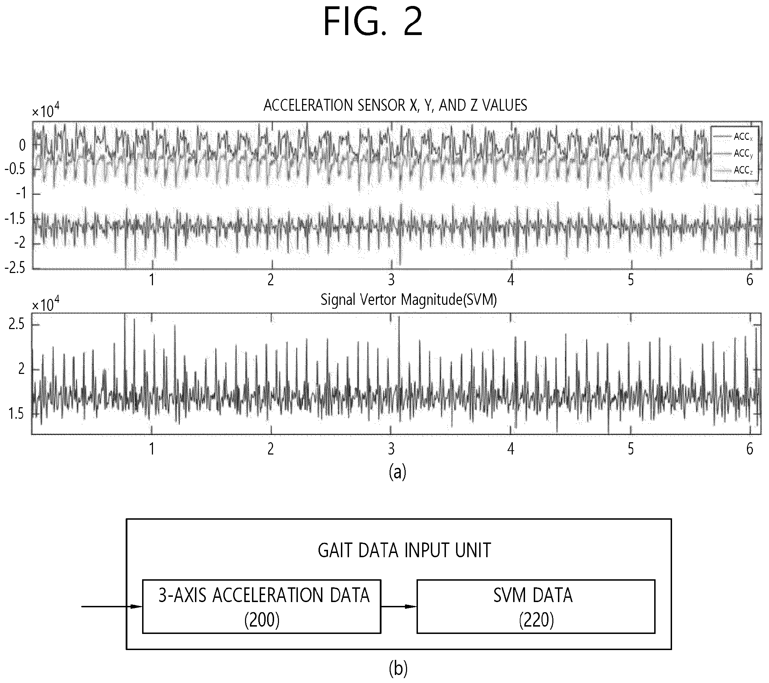

FIG. 2 shows conceptual diagrams showing gait data according to an embodiment of the present invention.

In FIG. 2, gait data used to prevent a user from falling is disclosed.

Referring to FIG. 2, the gait data may be determined based on acceleration signals obtained while the user is walking. The gait data may be later used to determine a gait speed and balance of both feet of the user.

The gait data may be signal vector magnitude (SVM) data on each of x-, y-, and z-axis acceleration signals. SVM data 220 is data obtained by offsetting direction components of 3-axis acceleration data 200 with respect to gravity and extracting a magnitude of a signal vector. The SVM data 220 may be determined regardless of the mixed loss in the axial direction of the sensor.

Equation 1 below is an equation for determining the SVM data 220.

<Equation 1>

$$\mathrm{SVM} = \sqrt{ACC_x^2 + ACC_y^2 + ACC_z^2}$$

$ACC_x$ denotes an x-axis acceleration value, $ACC_y$ denotes a y-axis acceleration value, and $ACC_z$ denotes a z-axis acceleration value.

FIG. 2A shows the 3-axis acceleration data 200 and the SVM data 220 that is determined based on the 3-axis acceleration data 200. The gait data represented by the SVM data 220 may be transmitted to and analyzed by a gait data analysis unit.

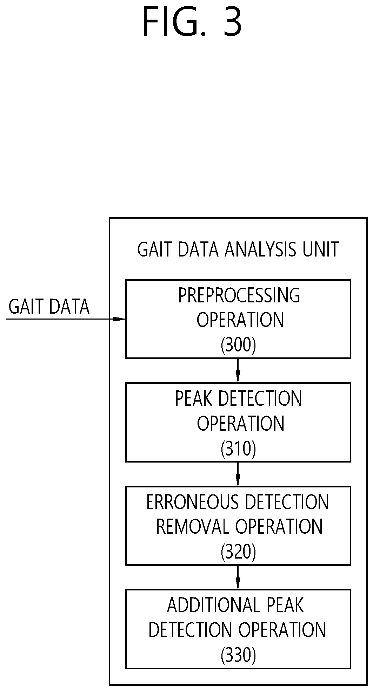

FIG. 3 is a conceptual diagram showing an operation of a gait data analysis unit according to an embodiment of the present invention.

In FIG. 3, a preprocessing operation of the gait data analysis unit to be performed on gait data is disclosed.

Referring to FIG. 3, the gait data analysis unit may determine a gait step of the user by performing a preprocessing operation 300, a peak detection operation 310, an erroneous detection removal operation 320, and an additional peak detection operation 330 on the basis of gait data. Thereafter, the gait data analysis unit may determine a gait speed on the basis of the determined gait step and information on the user's body.

Further, the gait data analysis unit determines an impulse on each foot on the basis of a difference in amplitude between a maximum peak (or upper peak) and a minimum peak (or lower peak) that are generated when one foot and the other foot touch the ground in the gait data obtained from a 3-axis acceleration sensor, and the gait data analysis unit may determine the balance of both feet on the basis of the impulses on both feet.

Hereinafter, a detailed operation of the gait data analysis unit is disclosed.

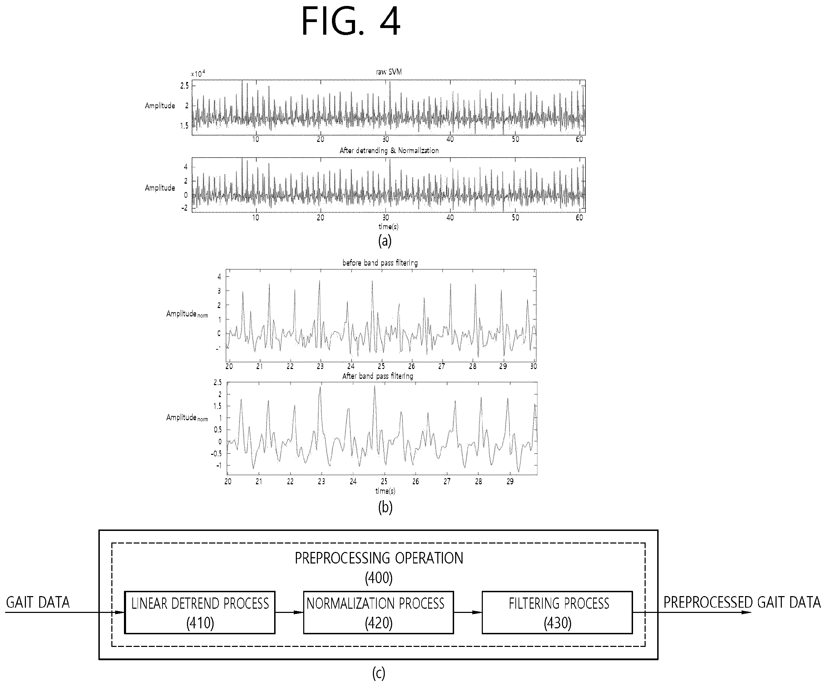

FIG. 4 shows conceptual diagrams showing the operation of the gait data analysis unit according to the embodiment of the present invention.

In FIG. 4, the preprocessing operation of the gait data analysis unit to be performed on gait data is disclosed.

Referring to FIG. 4, in a preprocessing operation 400, a linear detrend process 410 and a normalization process 420 for subtracting a mean value from the gait data may be preferentially performed.

The linear detrend process 410 may be used to extract the gait data from which a linear component is removed. The normalization process 420 is a process for matching ranges of a large amount of different gait data or making distributions similar, and the normalization process 420 may be performed through a process of dividing a value obtained by subtracting a mean from the gait data by variance. A difference of 1 when the distributions are similar is different from a difference of 1 when the distribution of values is very large. Therefore, in the present invention, the value obtained by subtracting a mean from the gait data is divided by the variance through the normalization process 420, and thus an effect due to the spread of the original distribution may be offset.

FIG. 4A is a graph of the gait data on which the linear detrend process 410 and the normalization process 420 are performed. A change in amplitude on a y-axis with respect to the gait data may be confirmed.

In the preprocessing operation 400, the linear detrend process 410 and the normalization process 420 are performed on the gait data, and then a filtering process 430 may be performed. A band-pass filter (e.g., a fourth-order finite impulse response (FIR) band-pass filter) having a cutoff frequency (e.g., 0.5 to 5 Hz) may be applied to the gait data, and thus the gait data may be processed to more easily detect the peaks in the peak detection operation later.

FIG. 4B shows preprocessed gait data on which the linear detrend process 410 and the normalization process 420 are performed and then the filtering process 430 is applied. The preprocessed gait data is data from which the remaining highfrequency components except for main peaks are removed. The gait data after the preprocessing operation may be expressed as a term of preprocessed gait data.

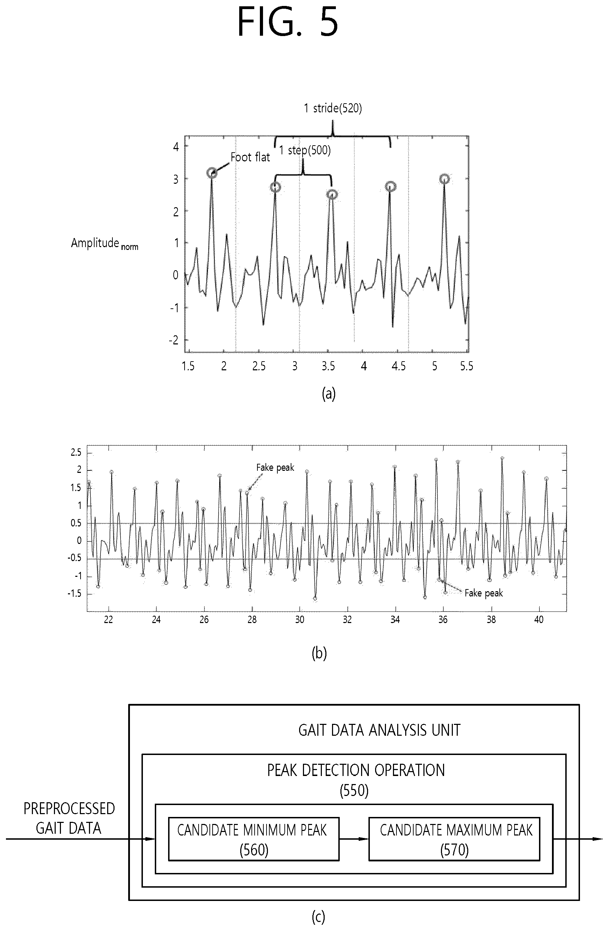

FIG. 5 shows conceptual diagrams showing the operation of the gait data analysis unit according to the embodiment of the present invention.

In FIG. 5, the peak detection operation, which is performed after the preprocessing operation of the gait data analysis is performed on the gait data, is disclosed.

Referring to FIG. 5, in a peak detection operation 550, peak detection may be performed on the preprocessed gait data for gait data analysis.

The peak detection may be performed in units of 1 step 500, and a section from one peak to the next peak and then the next peak after that may be defined as 1 stride 520. That is, the 1 stride 520 may include 2 step units.

Referring to FIG. 5A, since a total of five peaks are generated in the preprocessed gait data, a total of five steps may be calculated.

In the present invention, peaks for determining a gait step and an impulse may be a minimum peak 560 and a maximum peak 570. In the peak detection operation 550, an operation for detecting a candidate minimum peak 560 and a candidate maximum peak 570 is performed. The candidate minimum peak 560 and the candidate maximum peak 570 which are detected in the peak detection operation 550 may be peaks before fake peaks are removed.

The candidate maximum peak 570 and the candidate minimum peak 560 may be detected by setting lower and upper thresholds (e.g., 0.5) and finding local maxima and local minima from among the preprocessed gait data having a value greater than or equal to the thresholds.

FIG. 5B shows a result obtained by detecting the candidate maximum peak 570 and the candidate minimum peak 560 using the threshold setting method. Since there are several peaks in one gait period, it is difficult to find exactly one peak using only the local maxima and minima. As shown in FIG. 5B, fake peaks may be detected. In order to remove the detected fake peaks, an erroneous detection removal operation is required.

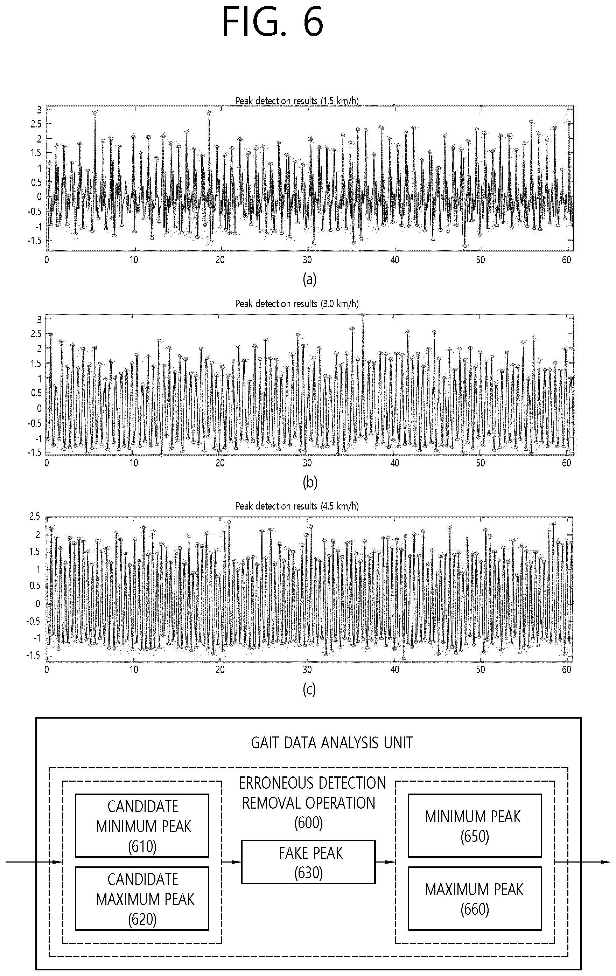

FIG. 6 shows conceptual diagrams showing an erroneous detection removal operation according to an embodiment of the present invention.

In FIG. 6, a method of performing the erroneous detection removal operation, which is performed after the preprocessing operation and the peak detection operation of the gait data analysis unit are performed on the gait data, is disclosed.

Referring to FIG. 6, in an erroneous detection removal operation 600, in order to remove a fake peak 630 from a candidate minimum peak 610 and a candidate maximum peak 620, a process in which a minimum one gait period is set as a critical period (e.g., 0.3 seconds) and information on peaks generated within the critical period is removed after one peak is generated may be performed. The fake peak 630 among the candidate minimum peak 610 and the candidate maximum peak 620 which are detected in the peak detection operation rare removed through the erroneous detection removal operation 600, and finally, a minimum peak 650 and a maximum peak 660 rare determined.

As results obtained by detecting the peaks after the removing of the fake peak 630, FIG. 6A shows a result obtained by detecting a peak in slow walking (1.5 km/h), FIG. 6B shows a result obtained by detecting a peak in normal walking (3.0 km/h), and FIG. 6C shows a result obtained by detecting a peak in fast walking (4.5 km/h).

Referring to FIGS. 6A, 6B, and 6C, as compared with the previous result, it can be confirmed that only one peak is accurately detected. That is, the maximum peak and the minimum peak may be accurately detected at all gait speeds.

In the present invention, the erroneous detection removal operation 600 may be adaptively performed according to the gait speed. When the gait speed is relatively slow, relatively more peaks are generated due to the generation of several frequency components. Therefore, in order to prevent such erroneous detection of peaks, artificial-intelligence-based peak extraction learning modeling according to a gait speed may be generated, and fake peaks may be adaptively removed according to the gait speed.

The gait data analysis unit may determine a gait step on the basis of the maximum peak 660 and the minimum peak 650 after the fake peaks are removed through the erroneous detection removal operation 600.

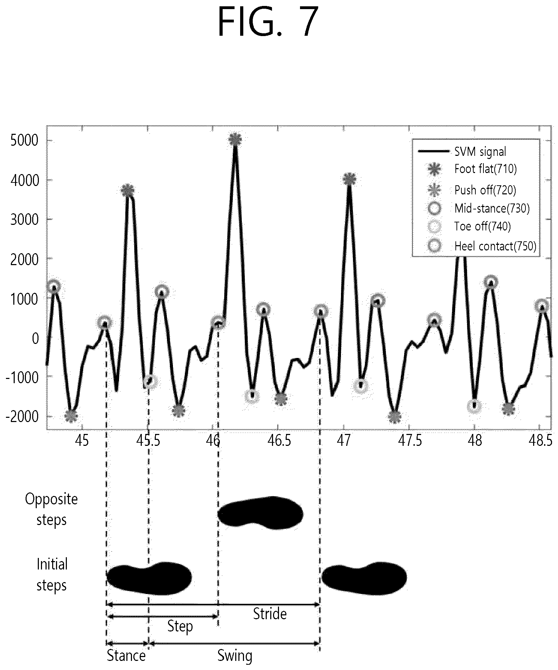

FIG. 7 is a conceptual diagram showing an additional peak detection operation according to an embodiment of the present invention.

In FIG. 7, the method of performing the erroneous detection removal operation, which is performed after the preprocessing operation and the peak detection operation of the gait data analysis unit are performed on the gait data, is disclosed.

Referring to FIG. 7, information on the gait step and information on the impulse may be obtained using the maximum peak. The information on the impulse and information on the balance of both feet is obtained using the maximum peak and the minimum peak.

Information on additional gait characteristics may be extracted based on a plurality of additional peaks, which are generated in the gait data, except for the maximum peak and the minimum peak.

The additional peaks may be divided by one waveform on the basis of the extracted maximum peak and then additionally extracted within the divided section. Through such an additional peak extraction process, n additional peaks (e.g., n=3) may be extracted in the segmented section.

As a result, in gait speed data of 1.5 km/h, five feature points such as a foot flat 710, a push-off 720, a toe-off 740, a mid-stance 730, and a heel contact 750 may be detected as the maximum peak, the minimum peak, and the additional peaks. The five feature points may be used to extract additional gait features such as a swing phase and a stance phase. The additional peaks may be used to reduce the likelihood of the user falling by additionally determining the gait characteristics of the user.

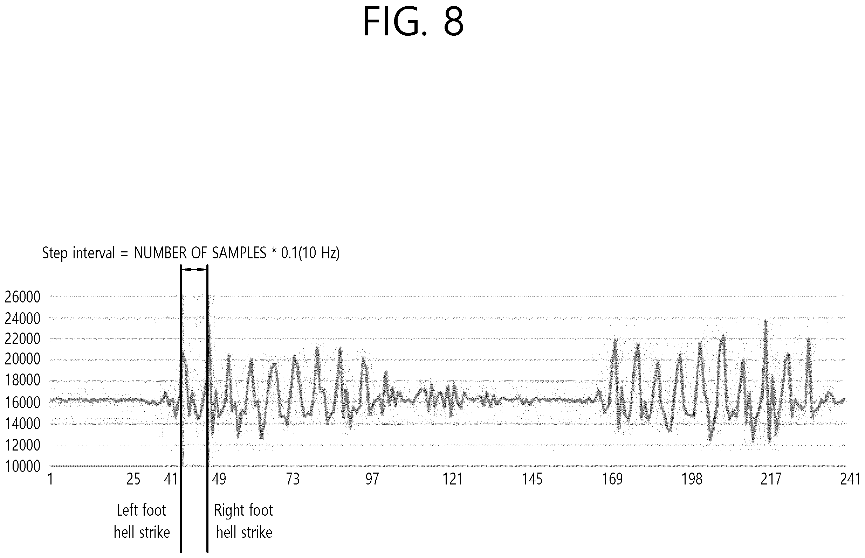

FIG. 8 is a conceptual diagram showing a method of determining, by a gait data analysis unit, a gait speed according to an embodiment of the present invention.

In FIG. 8, the method of determining, by a gait data analysis unit, the gait speed after maximum peaks are detected is disclosed.

Referring to FIG. 8, a gait step may be determined through detection of the maximum peaks, and a continuous step interval may be determined from real-time gait data.

In FIG. 8, in order to calculate the gait speed on the basis of the gait data, gait acceleration data when starting with a left foot, walking 20 m, then turning back and walking 20 m is exemplarily disclosed.

A maximum peak that is generated first is defined as a left foot and a maximum peak that is generated next is defined as a right foot, and a step interval time may be determined by multiplying the number of samples between steps by an acceleration sensor coefficient (e.g., 0 or 1).

Specifically, x-, y-, and z-axis acceleration signals that determine the gait data may be collected at 10 Hz (10 times per second), and ten pieces of gait data may be generated per second. In the case in which a peak-to-peak sample size is six, in consideration of the fact that six pieces of acceleration data are collected, 6*0.1=0.6 seconds, that is, 1 step time from a time the left foot is placed on the ground to a time the right foot touches the ground, may be regarded as 0.6 seconds, and it can be determined that the step interval time is 0.6 seconds.

Since the step interval time is determined, when a moving distance is determined through the steps, the gait speed may be determined by dividing a step length by the step interval time.

As the method of determining the gait speed, methods using height data or other pieces of body data (e.g., hip acceleration data) may be used. In the present invention, for convenience of description, a method of determining a gait speed using height data is disclosed.

The method of determining the gait speed using the height data has a small amount of computation and is simple, and thus the method makes it easy to collect long-term data. A method of determining a gait speed using hip acceleration data is a regression formula application method in which a change in center of mass of a vertical axis of an acceleration sensor is used as a variable, and thus the method makes it easy to collect short-term data.

In the present invention, step length estimation is performed based on the height data. The gait speed may be determined by dividing the estimated step length by the step interval time. The method of performing the step length estimation using the height data may be performed by applying a regression analysis result according to sex which is calculated based on a plurality of subjects.

Specifically, in the method of performing the step length estimation using the height data, a stride length may be determined by applying the regression analysis result according to sex calculated based on the plurality of subjects.

Equation 2 below shows equations for calculating male/female stride lengths based on the regression analysis result.

$$\text{<Equation 2>}$$

$$\text{Male}=(0.415 \times \text{height})$$

$$\text{Female}=(0.413 \times \text{height})$$

The step length used in the present invention may be calculated by multiplying the stride length by a step coefficient (e.g., 1/2). The step coefficient may be a value having a correlation in a critical confidence interval for a relationship between the stride length and the step length.

When the step length is calculated, the gait data analysis unit may determine the gait speed by dividing the step length by the step interval time.

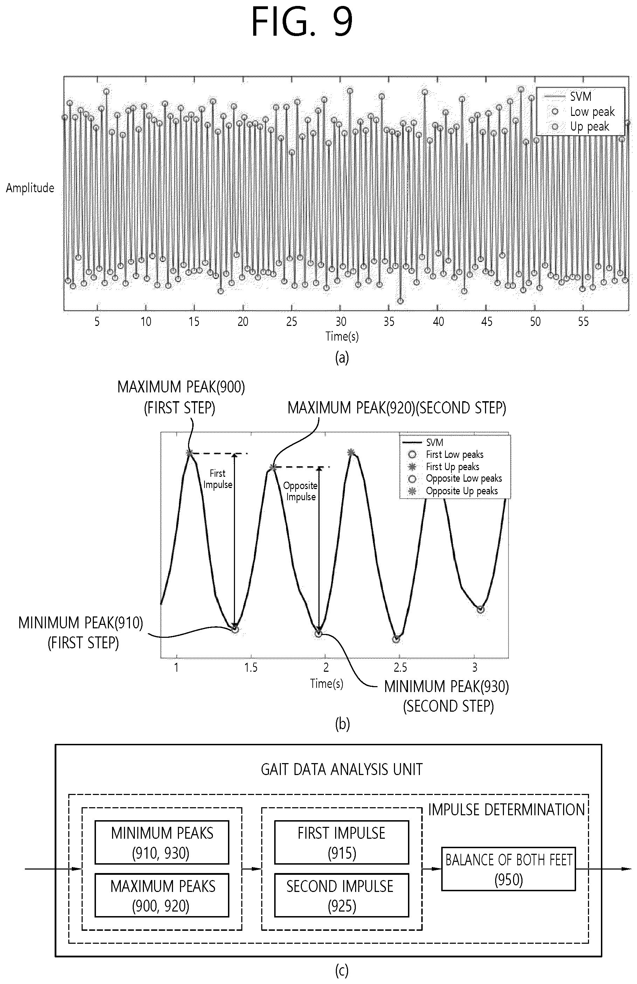

FIG. 9 shows conceptual diagrams showing a method of determining, by the gait data analysis unit, an impulse according to the embodiment of the present invention.

In FIG. 9, the method of determining, by a gait data analysis unit, the impulse is disclosed.

Referring to FIG. 9, the gait data analysis unit may determine an impulse on the basis of a difference in amplitude between continuous maximum peaks 900 and 920 and continuous minimum peaks 910 and 930 that are generated while the user is walking.

FIG. 9A shows gait data obtained for one minute on a treadmill at a 0° slope and 3.0 km/h speed as exemplary data for determining the impulse.

In FIG. 9B, in the gait data, a peak that is first generated rare defined as a first step, a peak that is generated immediately after the first peak is defined as a second step (opposite step), and impulses on the successive first and second steps are calculated and stored.

The following is a description of data obtained by calculating impulses for nine subjects.

The experiment was performed three times each for nine subjects, and a plurality of first impulses 915 on a plurality of first steps and a plurality of second impulses 925 on a plurality of second steps, which are calculated from a total of 27 one-minute walking signals, may be collected. A mean value of the plurality of first impulses 915 and a mean value of the plurality of second impulses 925 are determined.

Balance 950 of both feet is determined by dividing the relatively larger mean value among the mean value of the plurality of first impulses 915 and the mean value of the plurality of second impulses 925 by the relatively smaller mean value.

As the balance 950 of both feet approaches 1, the impulses 915 and 925 of both feet are similar, and thus it is determined that balance of the body is good. As the balance 950 of both feet is further away from 1, the impulses 915 and 925 of both feet are different, and thus it is determined that the balance of the body is not good.

<Table 1>

Impulse values (mean)

| Trial | Step | Subject | | | | | | | | |
|---|---|---|---|---|---|---|---|---|---|---|
| | | 1 | 2 | 3 | 4 | 5 | 6 | 7 | 8 | 9 |
| 1 | first step | 5.418 | 7.225 | 6.073 | 5.392 | 5.613 | 6.409 | 6.543 | 6.44 | 6.686 |
| | opposite step | 6.298 | 7.125 | 6.703 | 4.869 | 5.323 | 6.480 | 6.914 | 6.403 | 6.443 |
| | balance | 1.162 | 1.014 | 1.104 | 1.108 | 1.054 | 1.011 | 1.057 | 1.006 | 1.038 |
| 2 | first step | 5.409 | 7.222 | 6.027 | 4.872 | 5.594 | 6.354 | 6.821 | 6.481 | 6.822 |
| | opposite step | 6.235 | 7.044 | 6.999 | 5.653 | 5.225 | 6.691 | 6.434 | 6.647 | 6.345 |
| | halance | 1.153 | 1.025 | 1.161 | 1.160 | 1.071 | 1.053 | 1.060 | 1.026 | 1.075 |
| 3 | first step | 5.481 | 7.126 | 5.842 | 5.195 | 5.777 | 6.596 | 6.394 | 6.472 | 6.565 |
| | opposite step | 6.191 | 7.325 | 6.853 | 5.643 | 5.090 | 6.719 | 6.903 | 6.641 | 6,131 |
| | balance | 1.129 | 1.029 | 1.173 | 1.086 | 1.135 | 1.019 | 1.080 | 1.026 | 1.071 |

(continued)

Impulse values (mean)

| Trial | Step | Subject | | | | | | | | |
|---|---|---|---|---|---|---|---|---|---|---|
| | | 1 | 2 | 3 | 4 | 5 | 6 | 7 | 8 | 9 |
| | first step | 5.436 | 7.191 | 5.981 | 5.153 | 5.661 | 6.453 | 6.586 | 6.464 | 6.691 |
| mean | opposite step | 6.241 | 7.165 | 6.852 | 5.388 | 5.213 | 6.630 | 6.750 | 6.564 | 6.306 |
| | balance | 1.148 | 1.023 | 1.146 | 1.118 | 1.087 | 1.028 | 1.066 | 1.019 | 1.061 |

The gait data analysis unit may determine information on the impulses 915 and 925 and the balance 950 of both feet and transmit the information to the fall estimation unit.

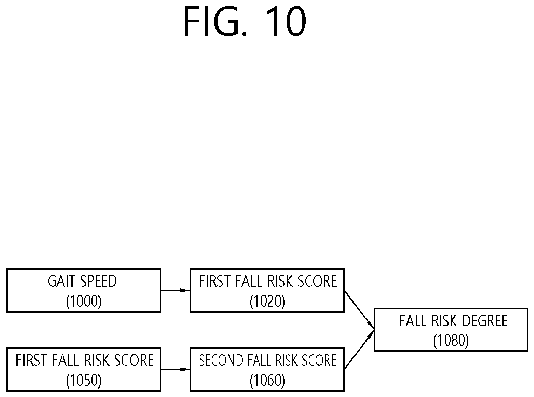

FIG. 10 is a conceptual diagram showing a method of estimating, by the fall estimation unit, a fall according to an embodiment of the present invention.

**In** FIG. 10, a method of estimating, by the fall estimation unit, a fall risk based on the gait speed and the balance of both feet which are transmitted by the gait data analysis unit is disclosed.

Referring to FIG. 10, a fall risk degree 1080 may be determined by additionally considering balance 1050 of both feet and a gait speed 1000.

**In** the present invention, the balance 1050 of both feet may be expressed as distribution information for a specific speed. For example, when the gait speed 1000 of the user is x m/s, the balance 1050 of both feet may be expressed as a distribution when the user is walking at x m/s. For example, when it is assumed that the balance 1050 of both feet with the same impulse on the right foot and the left foot is 0, the balance 1050 of both feet may be set to have a negative value when the balance is shifted to the left foot based on 0, and to have a positive value when the balance is shifted to the right foot based on 0.

The fall risk degree may be determined as a numerical value based on the balance 1050 of both feet and the gait speed 1000. A score, the balance 1050 of both feet for determining the score, and a range of the gait speed 1000 are exemplary and may vary.

Table 2 below shows a first fall risk score 1020 according to the gait speed 1000.

<Table 2>

| Gait speed (m/s) | Risk degree | Score |
|---|---|---|
| 1.2 <= x | 0.00% | **10** |
| 1 <= x < 1.2 | 6.25% | **9** |
| 0.9 <= x < 1 | 12.50% | **8** |
| 0.8 <= x < 0.9 | 25% | **7** |
| 0.7 <= x < 0.8 | 37.50% | **6** |
| 0.6 <= x < 0.7 | 55.50% | **5** |
| 0.5 <= x < 0.6 | 73.50% | **4** |
| 0 < x < 0.5 | Very high | **3** |

As the gait speed 1000 decreases, the fall risk degree may be relatively high and a lower score may be assigned.

Table 3 below shows a second fall risk score 1060 according to the balance 1050 of both feet.

<Table 3>

| abs(RFP-LFP)/max(RFP,LFP) | Score |
|---|---|
| x < 0.1 | **0** |
| 0.1 <= x < 0.2 | **-1** |
| 0.2 <= x < 0.3 | **-2** |
| x >= 0.3 | **-3** |

RFP denotes the impulse on the right foot, and LFP denotes the impulse on the left foot. The second fall risk score 1060 may be determined based on a value obtained by dividing an absolute value of a value obtained by subtracting the impulse on the left foot from the impulse on the right foot, or abs(RFP-LFP), by a maximum value among the impulse on the right foot and the impulse on the left foot, or max(RFP, LFP).

Table 4 below shows the fall risk degree 1080 based on the first fall risk score 1020 and the second fall risk score 1060.

<Table 4>

| Total score | Fall risk degree | Lotti |
|---|---|---|
| 10 | Very low | Fall_10 |
| 9 | | Fall_9 |
| 8 | Low | Fall_8 |
| 7 | | Fall_7 |
| 6 | Medium | Fall_6 |
| 5 | | Fall_5 |
| 4 | High | Fall_1~4 |
| 3 | | |
| 2 | | |
| 1 | Very high | |
| 0 | | Stand_hi |

A total score for estimating the likelihood of falling may be the sum of the first fall risk score 1020 and the second fall risk score 1060. The total score for estimating the likelihood of falling may be a value from 0 to 10, and it may be determined that the fall risk degree 1080 increases as the total score decreases.

Further, in the present invention, in addition to the likelihood of falling, information on the balance 1050 of both feet may be provided to the user as evaluation information on the balance of both feet, size information on the balance of both feet, and deviation information on the balance of both feet.

The evaluation information on the balance of both feet may be provided as shown in Table 5 below.

<Table 5>

| \|RFP−LFP\| | Score | UI text |
|---|---|---|
| x < 1 | 5 | GOOD |
| 1 <= x < 2 | 4 | FAIR |
| 2 <= x < 3 | 3 | WORRIED |
| 3 <= x < 4 | 2 | POOR |
| x >= 4 | 1 | BAD |

The balance of both feet may be evaluated based on an absolute value of a difference between the RFP and the LFP and may be provided in five levels (GOOD, FAIR, WORRIED, POOR, and BAD).

The deviation information on the balance of both feet may be provided as shown in Table 6 below.

<Table 6>

| RFP-LFP | Left and right reference positions |
|---|---|
| -4 | Right max. |
| -3 | 3-4 |
| -2 | 2-4 |

(continued)

| RFP-LFP | Left and right reference positions |
|---|---|
| -1 | 1-4 |
| 0 | Central axis |
| 1 | 1-4 |
| 2 | 2-4 |
| 3 | 3-4 |
| 4 | Left max. |

A degree to which the balance is shifted to the left foot or the right foot with respect to the center of the difference value may be provided based on the deviation information on the balance of both feet.

The size information on the balance of both feet may be provided as shown in Table 7 below.

<Table 7>

| Power avg. (LFP, RFP) | Size of circle |
|---|---|
| x < 5 | 1/6 |
| 5 <= x < 7 | 2/6 |
| 7 <= x < 9 | 3/6 |
| 9 <= x < 11 | 4/6 |
| 11 <= x < 13 | 5/6 |
| 13 <= x | 6/6 |

Based on a mean value of the impulses on the left foot and the right foot, information on the impulses generated in both feet may be provided based on the size information on the balance of both feet.

Further, according to the embodiment of the present invention, information on each of the 3-axis accelerations that generate the above-described gait data may be used for fall estimation.

When an x-axis acceleration is called $A_x(t)$, a y-axis acceleration is called $A_y(t)$, and a z-axis acceleration is called $A_z(t)$, a fall may be estimated more accurately based on a magnitude of movement by eliminating an effect of gravity.

Therefore, the fall estimation may be performed using Equation 3 below considering only a weight in a direction of gravity as shown below.

<Equation 3>

$$\theta(t) = \tan^{-1}\left(\frac{A_x^2(t)}{\sqrt{A_y^2(t) + A_z^2(t)}}\right)$$

$$\psi(t) = \tan^{-1}\left(\frac{A_z^2(t)}{\sqrt{A_x^2(t) + A_y^2(t)}}\right)$$

$$A_{DSVM}(t) = \sqrt{(A_x(t) - A_x(t-1))^2 + (A_y(t) - A_y(t-1))^2 + (A_z(t) - A_z(t-1))^2}$$

$$A_{\theta GDSVM}(t) = \left(\frac{\theta_{mean}(t)}{90} \times A_{DSVM}(t)\right)$$

$$A_{\psi GDSVM}(t) = \left(\frac{\psi_{mean}(t)}{90} \times A_{DSVM}(t)\right)$$

$\theta(t)$ denotes a left/right angle formed with the direction of gravity, and $\psi(t)$ denotes a forward/rearward angle formed with the direction of gravity. $A_{DSVM}(t)$ denotes an amount of changes in acceleration. $A_{\theta GDSVM}(t)$ denotes a value obtained by amplifying a signal on the basis of $\theta(t)$ when the user is falling, and $A_{\psi GDSVM}(t)$ denotes a value obtained by amplifying a signal on the basis of $\psi(t)$ when the user is falling.

When one of $A_{\theta GDSVM}(t)$ and $A_{\psi GDSVM}(t)$ exceeds a threshold value, a warning of the likelihood of falling may be provided to the user, when $A_{\theta GDSVM}(t)$ is greater than the threshold value, a warning of the likelihood of falling forward/rearward may be provided to the user, and when $A_{\psi GDSVNM}(t)$ is greater than the threshold value, a warning of the likelihood of falling left/right may be provided to the user.

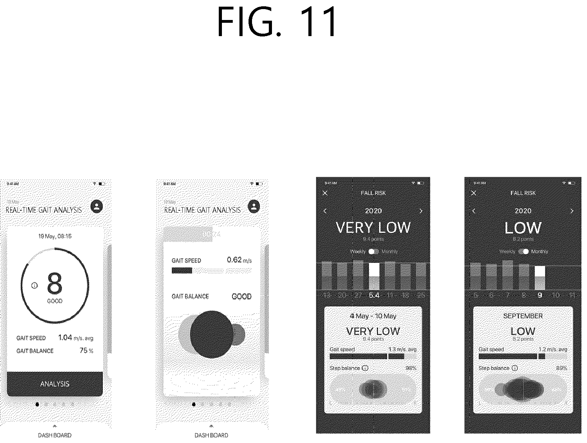

FIG. 11 shows conceptual diagrams showing a user screen for displaying results of estimating falls according to an embodiment of the present invention.

In FIG. 11, the user screen, on which fall estimation results are provided, is disclosed.

Referring to FIG. 11, information on a total score for estimating the likelihood of falling may be provided on a first screen.

Evaluation information on balance of both feet, size information on the balance of both feet, and deviation information on the balance of both feet may be provided on a second screen.

The information on the total score for estimating the likelihood of falling, the evaluation information on the balance of both feet, the size information on the balance of both feet, and the deviation information on the balance of both feet may be provided on a third screen and a fourth screen.

According to the present invention, by collecting gait data of a user, a fall risk that may occur for the user can be estimated, and thus falls can be prevented.

Further, according to the present invention, by performing preprocessing of the gait data of the user, a fall risk that may occur for the user can be estimated more accurately in consideration of a gait speed of the user, and thus falls can be prevented.

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the scope of the present invention are defined not by the detailed description of the present invention but by the appended claims, and encompass all modifications and equivalents that fall within the scope of the appended claims.

**Claims**

1. A method of preventing a fall risk of a user, the method comprising:

receiving, by a fall prevention apparatus, gait data;
generating, by the fall prevention apparatus, gait analysis data on the basis of analysis of the gait data; and
generating, by the fall prevention apparatus, fall prevention data on the basis of the gait analysis data, wherein:

analysis of the gait data is performed based on a maximum peak and a minimum peak appearing in the gait data; and
the gait analysis data includes data on a gait speed and balance of both feet (950), **characterized in that** the gait speed is determined based on a step interval time determined based on the maximum peak;
wherein the balance of both feet (950) is determined using a plurality of first impulses (915) calculated based on an amplitude between the maximum peak and the minimum peak on a plurality of successive first steps, and using a plurality of second impulses (925) calculated based on an amplitude between the maximum peak and the minimum peak on a plurality of successive second steps, the second steps being opposite the first steps, wherein the balance of both feet is determined by dividing the relatively larger mean value among a mean value of the plurality of first impulses and a mean value of the plurality of second impulses by the relatively smaller mean value, and
wherein the maximum and minimum peaks (650, 660) are determined by using an erroneous detection removal operation (320, 600) to remove a fake peak (630) from a candidate minimum peak (610) and a candidate maximum peak (620), in which erroneous detection removal operation (320, 600) a minimum one gait period is set as a critical period and information on peaks generated within the critical period is removed after one peak is generated.

2. A fall prevention apparatus for preventing a fall risk of a user, the apparatus comprising:

a gait data input unit configured to receive gait data;
a gait data analysis unit configured to generate gait analysis data on the basis of analysis of the gait data; and
a fall prevention unit configured to generate fall prevention data on the basis of the gait analysis data, wherein:

analysis of the gait data is performed based on a maximum peak and a minimum peak appearing in the gait data; and
the gait analysis data includes data on a gait speed and balance of both feet, **characterized in that** the gait speed is determined based on a step interval time determined based on the maximum peak; and
the balance of both feet is determined using a plurality of first impulses calculated based on an amplitude between the maximum peak and the minimum peak on a plurality of successive first steps, and using a plurality of second impulses (925) calculated based on an amplitude between the maximum peak and the minimum peak on a plurality of successive second steps, the second steps being opposite the first steps, wherein the balance of both feet is determined by dividing the relatively larger mean value among a mean value of the plurality of first impulses and a mean value of the plurality of second impulses by the relatively smaller mean value, and
wherein the maximum and minimum peaks (650, 660) are determined by using an erroneous detection removal operation (320, 600) to remove a fake peak (630) from a candidate minimum peak (610) and a candidate maximum peak (620), in which erroneous detection removal operation (320, 600) a minimum one gait period is set as a critical period and information on peaks generated within the critical period is removed after one peak is generated.

**Patentansprüche**

1. Verfahren zum Verhindern des Sturzrisikos eines Benutzers, wobei das Verfahren Folgendes umfasst:

Empfangen von Gangdaten durch eine Sturzverhinderungsvorrichtung;
Erzeugen von Ganganalysedaten durch die Sturzverhinderungsvorrichtung auf der Basis einer Analyse der Gangdaten; und
Erzeugen von Sturzverhinderungsdaten durch die Sturzverhinderungsvorrichtung auf der Basis der Ganganalysedaten, wobei:

die Analyse der Gangdaten basierend auf einem maximalen Spitzenwert und einem minimalen Spitzenwert durchgeführt wird, die in den Gangdaten auftreten; und
die Ganganalysedaten Daten über eine Ganggeschwindigkeit und ein Gleichgewicht beider Füße (950) aufweisen,
**dadurch gekennzeichnet, dass**
die Ganggeschwindigkeit basierend auf einer Schrittintervallzeit bestimmt wird, die basierend auf dem maximalen Spitzenwert bestimmt wird;
wobei das Gleichgewicht beider Füße (950) unter Verwendung einer Mehrzahl erster Impulse (915), die basierend auf einer Amplitude zwischen dem maximalen Spitzenwert und dem minimalen Spitzenwert bei einer Mehrzahl aufeinanderfolgender erster Schritte berechnet wird, und unter Verwendung einer Mehrzahl zweiter Impulse (925) bestimmt wird, die basierend auf einer Amplitude zwischen dem maximalen Spitzenwert und dem minimalen Spitzenwert bei einer Mehrzahl aufeinanderfolgender zweiter Schritte berechnet wird, wobei die zweiten Schritte gegenüber den ersten Schritten sind, wobei das Gleichgewicht beider Füße durch Teilen des relativ größeren Mittelwerts zwischen einem Mittelwert der Mehrzahl erster Impulse und einem Mittelwert der Mehrzahl zweiter Impulse durch den relativ kleineren Mittelwert bestimmt wird, und wobei der maximale und der minimale Spitzenwert (650, 660) durch Verwenden einer Operation zur Entfernung fehlerhafter Detektionen (320, 600) bestimmt werden, um einen falschen Spitzenwert (630) aus einem in Frage kommenden minimalen Spitzenwert (610) und einem in Frage kommenden maximalen Spitzenwert (620) zu entfernen, wobei in der Operation zur Entfernung fehlerhafter Detektionen (320, 600) mindestens eine Gangperiode als eine kritische Periode festgelegt wird und Informationen über Spitzenwerte, die innerhalb der kritischen Periode erzeugt werden, entfernt werden, nachdem ein Spitzenwert erzeugt wurde.

2. Sturzverhinderungsvorrichtung zum Verhindern des Sturzrisikos eines Benutzers, wobei die Vorrichtung Folgendes umfasst:

eine Gangdateneingabeeinheit, die dazu ausgelegt ist, Gangdaten zu empfangen;
eine Gangdatenanalyseeinheit, die dazu ausgelegt ist, Ganganalysedaten auf der Basis einer Analyse der Gangdaten zu erzeugen; und
eine Sturzverhinderungseinheit, die dazu ausgelegt ist, Sturzverhinderungsdaten auf der Basis der Ganganalysedaten zu erzeugen, wobei:

die Analyse der Gangdaten basierend auf einem maximalen Spitzenwert und einem minimalen Spitzenwert durchgeführt wird, die in den Gangdaten auftreten; und
die Ganganalysedaten Daten über eine Ganggeschwindigkeit und ein Gleichgewicht beider Füße umfassen,
**dadurch gekennzeichnet, dass**
die Ganggeschwindigkeit basierend auf einer Schrittintervallzeit bestimmt wird, die basierend auf dem maximalen Spitzenwert bestimmt wird; und
wobei das Gleichgewicht beider Füße unter Verwendung einer Mehrzahl erster Impulse, die basierend auf einer Amplitude zwischen dem maximalen Spitzenwert und dem minimalen Spitzenwert bei einer Mehrzahl aufeinanderfolgender erster Schritte berechnet wird, und unter Verwendung einer Mehrzahl zweiter Impulse (925) bestimmt wird, die basierend auf einer Amplitude zwischen dem maximalen Spitzenwert und dem minimalen Spitzenwert bei einer Mehrzahl aufeinanderfolgender zweiter Schritte berechnet wird, wobei die zweiten Schritte gegenüber den ersten Schritten sind, wobei das Gleichgewicht beider Füße durch Teilen des relativ größeren Mittelwerts zwischen einem Mittelwert der Mehrzahl erster Impulse und einem Mittelwert der Mehrzahl zweiter Impulse durch den relativ kleineren Mittelwert bestimmt wird, und wobei der maximale und der minimale Spitzenwert (650, 660) durch Verwenden einer Operation zur Entfernung fehlerhafter Detektionen (320, 600) bestimmt werden, um einen falschen Spitzenwert (630) aus einem in Frage kommenden minimalen Spitzenwert (610) und einem in Frage kommenden maximalen Spitzenwert (620) zu entfernen, wobei in der Operation zur Entfernung fehlerhafter Detektionen (320, 600) mindestens eine Gangperiode als eine kritische Periode festgelegt wird und Informationen über Spitzenwerte, die innerhalb der kritischen Periode erzeugt werden, entfernt werden, nachdem ein Spitzenwert erzeugt wurde.

**Revendications**

1. Procédé de prévention d'un risque de chute d'un utilisateur, le procédé comprenant :

la réception, par un appareil de prévention de chute, de données de marche ;
la génération, par l'appareil de prévention de chute, de données d'analyse de marche sur la base d'analyse des données de marche ; et
la génération, par l'appareil de prévention de chute, de données de prévention de chute sur la base des données d'analyse de marche, dans lequel :

l'analyse des données de marche est réalisée sur la base d'une crête maximum et d'une crête minimum apparaissant dans les données de marche ; et
les données d'analyse de marche incluent des données sur une vitesse de marche et un équilibre des deux pieds (950), **caractérisé en ce que**
la vitesse de marche est déterminée sur la base d'un temps d'intervalle de pas déterminé sur la base de la crête maximum ;
dans lequel l'équilibre des deux pieds (950) est déterminé en utilisant une pluralité de premières impulsions (915) calculées sur la base d'une amplitude entre la crête maximum et la crête minimum durant une pluralité de premiers pas successifs, et en utilisant une pluralité de secondes impulsions (925) calculées sur la base d'une amplitude entre la crête maximum et la crête minimum durant une pluralité de seconds pas successifs, les seconds pas étant opposés aux premiers pas, dans lequel l'équilibre des deux pieds est déterminé en divisant la valeur moyenne relativement plus grande parmi une valeur moyenne de la pluralité de premières impulsions et une valeur moyenne de la pluralité de secondes impulsions par la valeur moyenne relativement plus petite, et
dans lequel les points maximum et minimum (650, 660) sont déterminés en utilisant une opération d'élimination de détection erronée (320, 600) pour éliminer un faux point (630) d'une crête minimum candidat (610) et d'une crête maximum candidat (620), dans laquelle opération d'élimination de détection erronée (320, 600) une période minimum d'une marche est réglée en tant que période critique et des informations sur des points générés dans les limites de la période critique sont éliminées après qu'une crête est générée.

2. Appareil de prévention de chute pour la prévention d'un risque de chute d'un utilisateur, l'appareil comprenant :

une unité d'entrée de données de marche configurée pour recevoir des données de marche ;
une unité d'analyse de données de marche configurée pour générer des données d'analyse de marche sur la base d'analyse des données de marche ; et
une unité de prévention de chute configurée pour générer des données de prévention de chute sur la base des données d'analyse de marche, dans lequel :

l'analyse des données de marche est réalisée sur la base d'une crête maximum et d'une crête minimum apparaissant dans les données de marche ; et
les données d'analyse de marche incluent des données sur une vitesse de marche et un équilibre des deux pieds,
**caractérisé en ce que**
la vitesse de marche est déterminée sur la base d'un temps d'intervalle de pas déterminé sur la base de la crête maximum ; et
l'équilibre des deux pieds est déterminé en utilisant une pluralité de premières impulsions calculées sur la base d'une amplitude entre la crête maximum et la crête minimum durant une pluralité de premiers pas successifs, et en utilisant une pluralité de secondes impulsions (925) calculées sur la base d'une amplitude entre la crête maximum et la crête minimum durant une pluralité de seconds pas successifs, les seconds pas étant opposés aux premiers pas, dans lequel l'équilibre des deux pieds est déterminé en divisant la valeur moyenne relativement plus grande parmi une valeur moyenne de la pluralité de premières impulsions et une valeur moyenne de la pluralité de secondes impulsions par la valeur moyenne relativement plus petite, et dans lequel les points maximum et minimum (650, 660) sont déterminés en utilisant une opération d'élimination de détection erronée (320, 600) pour éliminer un faux point (630) d'une crête minimum candidat (610) et d'une crête maximum candidat (620), dans laquelle opération d'élimination de détection erronée (320, 600) une période minimum d'une marche est réglée en tant que période critique et des informations sur des points générés dans les limites de la période critique sont éliminées après qu'une crête est générée.

# FIG. 1

```
┌─────────────────────────────────────────┐
│          FALL PREVENTION APPARATUS        │
│   ┌ ─ ─ ─ ─ ─ ─ ─ ─ ─ ─ ─ ─ ─ ─ ─ ─ ─ ┐   │
│   │  ┌─────────────────────────────┐  │   │
│   │  │    GAIT DATA INPUT UNIT     │  │   │
│   │  │           (100)             │  │   │
│   │  └─────────────────────────────┘  │   │
│   │  ┌─────────────────────────────┐  │   │
│   │  │   GAIT DATA ANALYSIS UNIT   │  │   │
│   │  │           (110)             │  │   │
│   │  └─────────────────────────────┘  │   │
│   │  ┌─────────────────────────────┐  │   │
│   │  │    FALL PREVENTION UNIT     │  │   │
│   │  │           (120)             │  │   │
│   │  └─────────────────────────────┘  │   │
│   └ ─ ─ ─ ─ ─ ─ ─ ─ ─ ─ ─ ─ ─ ─ ─ ─ ─ ┘   │
│                    ↕                      │
│   ┌─────────────────────────────────┐     │
│   │           PROCESSOR             │     │
│   │            (130)                │     │
│   └─────────────────────────────────┘     │
└─────────────────────────────────────────┘
```

# FIG. 2

ACCELERATION SENSOR X, Y, AND Z VALUES

Signal Vertor Magnitude(SVM)

(a)

GAIT DATA INPUT UNIT

| 3-AXIS ACCELERATION DATA (200) | SVM DATA (220) |

(b)

EP 4 198 933 B1

# FIG. 3

GAIT DATA ANALYSIS UNIT

GAIT DATA →

PREPROCESSING
OPERATION
(300)

↓

PEAK DETECTION
OPERATION
(310)

↓

ERRONEOUS DETECTION
REMOVAL OPERATION
(320)

↓

ADDITIONAL PEAK
DETECTION OPERATION
(330)

# FIG. 4

(a)

(b)

(c)

# FIG. 5

(a)

(b)

(c)

# FIG. 6

Peak detection results (1.5 km/h)

(a)

Peak detection results (3.0 km/h)

(b)

Peak detection results (4.5 km/h)

(c)

GAIT DATA ANALYSIS UNIT

CANDIDATE MINIMUM PEAK (610)

CANDIDATE MAXIMUM PEAK (620)

ERRONEOUS DETECTION REMOVAL OPERATION (600)

FAKE PEAK (630)

MINIMUM PEAK (650)

MAXIMUM PEAK (660)

# FIG. 7

# FIG. 8

Step interval = NUMBER OF SAMPLES * 0.1(10 Hz)

Left foot
hell strike

Right foot
hell strike

# FIG. 9

Amplitude

Time(s)

(a)

MAXIMUM PEAK(900)
(FIRST STEP)

MAXIMUM PEAK(920)(SECOND STEP)

First
Impulse

Opposite
Impulse

SVM
First Low peaks
First Up peaks
Opposite Low peaks
Opposite Up peaks

MINIMUM PEAK(910)
(FIRST STEP)

Time(s)

MINIMUM PEAK(930)
(SECOND STEP)

(b)

GAIT DATA ANALYSIS UNIT

| MINIMUM PEAKS (910, 930) | FIRST IMPULSE (915) | IMPULSE DETERMINATION |
|---|---|---|
| MAXIMUM PEAKS (900, 920) | SECOND IMPULSE (925) | BALANCE OF BOTH FEET (950) |

(c)

# FIG. 10

```
┌──────────────────┐      ┌──────────────────────┐
│   GAIT SPEED     │─────▶│ FIRST FALL RISK SCORE│──┐
│    (1000)        │      │       (1020)         │  │      ┌──────────────────┐
└──────────────────┘      └──────────────────────┘  ├─────▶│ FALL RISK DEGREE │
                                                     │      │     (1080)       │
┌──────────────────┐      ┌──────────────────────┐  │      └──────────────────┘
│FIRST FALL RISK   │─────▶│SECOND FALL RISK SCORE│──┘
│  SCORE (1050)    │      │       (1060)         │
└──────────────────┘      └──────────────────────┘
```

# FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160100776 A1
- US 20180177436 A1